# EUROPEAN PATENT SPECIFICATION

(11) **EP 1 539 060 B1**
(45) Date of publication and mention of the grant of the patent: **08.09.2010**
(21) Application number: 03771527.3
(22) Date of filing: 24.07.2003
(51) Int. Cl.: A61F 5/00, A61L 27/50

(54) **MULTI-MATERIAL CONSTRICTION DEVICE FOR FORMING STOMA OPENING**
KONSTRIKTIONSVORRICHTUNG AUS MEHREREN MATERIALIEN ZUR BILDUNG EINER STOMAÖFFNUNG
DISPOSITIF DE CONSTRICTION CONSTITUE DE MATIERES MULTIPLES POUR LA FORMATION D'UN ORIFICE DE STOMIE

(30) Priority: 29.07.2002 US 398810 P
(43) Date of publication of application: 15.06.2005
(73) Proprietor: Obesica AG, 6340 Baar (CH)
(72) Inventor: FORSELL, Peter, CH-6300 Zug (CH)
(74) Representative: Strandin, Heléne
(86) International application number: PCT/SE2003/001239
(87) International publication number: WO 2004/010910

(56) References cited:
- WO-A2-01/47435
- US-A- 4 428 365
- US-A- 4 756 949
- US-A- 5 873 904

## Description

The present invention relates to an implantable constriction device for forming a stoma opening in the stomach or esophagus of a patient, for treating obesity or reflux and heartburn disease.

This kind of constriction device, in the form of a gastric banding device, in which a band encircles and constricts a portion of a patient's stomach to restrict the food intake of the patient, has been used in surgery for morbid obesity to form a small gastric pouch above the band and a reduced stoma opening in the stomach. Although such a band is applied around the stomach to obtain an optimal stoma opening during surgery, some prior gastric banding devices are provided with an adjustment device enabling a minor post-operation adjustment of the size of the stoma opening. For example, U.S. Patent No. 6,067,991 discloses a mechanically adjusted gastric band and U.S. Patent No. 6,102,922 discloses a hydraulically adjusted gastric band having an inflatable cavity. In practice, the band is made of silicone, which is a material approved and widely used for implantation. Moreover, the silicone band has an acceptable tensile strength and is fairly resistant to aggressive body fluids. Where the band is hydraulically adjusted the hydraulic fluid used typically is an isotonic salt solution mixed with other conventional materials.

A problem with traditional silicone gastric bands, however, is that the silicone material gives the band certain inadequate properties, such as poor fatigue resistance and poor endurance of static bending forces, which over time might result in breakage of the band. Furthermore, silicone is a material that is semi-permeable by liquid, which is a drawback to hydraulic silicone bands, because hydraulic fluid can escape by diffusing through the silicone material. As a result, accurate adjustments of a hydraulic band are difficult to perform because of the loss of hydraulic fluid and the need for a patient to regularly visit a doctor to add hydraulic fluid to and calibrate the constriction device. These inadequate properties are serious, considering that the band is implanted for the rest of the patient's life. Another problem is that the band somewhat restrains the dynamics movements of the stomach necessary for digesting the food. As a consequence, the band might erode the stomach wall so that the stomach wall becomes thinner over time, which dramatically increases the risk of the band penetrating the stomach wall.

The kind of constriction device presented initially has also been used for treating heartburn and reflux disease due to hiatal hernia, i.e. a portion of the stomach immediately below the gastric fundus slides upwardly through the esophageal hiatus. A consequence of hiatal hernia is that stomach acids and foods are regurgitated into the esophagus. For example, WO 01/49245 A2 discloses a mechanically adjusted silicone band of a constriction device that constricts the esophagus (or the stomach close to the cardia) of a patient to close the esophagus between meals. Thus, in this case the constriction device functions as an artificial sphincter. WO 01/47435 A2 discloses a similar constriction device including a hydraulically adjusted silicone band also functioning as an artificial sphincter.

The same inadequate properties stated above in connection with gastric bands also apply for silicone bands designed for the treatment of heartburn and reflux disease. A specific problem of implanted bands for treating heartburn an reflux disease is that the movements of the esophagus occurring when the patient swallows food are somewhat restrained by the band. As a consequence, the band might erode and injure the esophagus.

The object of the present invention is to provide a new implantable constriction device for forming a stoma opening having improved properties as compared to traditional constriction devices.

Another object of the invention is to provide a new implantable constriction device suited for treating obese patients as well as patients suffering from heartburn and reflux disease.

Accordingly, the present invention provides an implantable constriction device for forming a restricted stoma opening in the stomach or esophagus of a patient according to claim 1.

In accordance with a first embodiment of the invention, the property improving means comprises a coating on the base material at least along a side of the elongate composite structure that is intended to contact the stomach or esophagus, wherein the coating has better aggressive body fluid resistance than the base material. Such a coating may comprise a Teflon^{™} or Parylene^{™} coating, or a biocompatible metal coating such as gold, silver or titanium. As a result, the constriction device can be protected from damaging influences of aggressive body fluids, possibly for the rest of the patient's life.

Where traditional silicone material constitutes the base material, a Teflon^{™} or Parylene^{™} coating also provides the composite structure with better anti-friction properties than the base material. Good anti-friction properties of the composite structure reduce the risk of the elongate composite structure eroding the stomach or esophagus. This is proven by tests, in which the surface of traditional silicone bands has been polished before use. Accordingly, the test results indicate significant improvements in avoiding erosion of the stomach or esophagus.

Furthermore, the Teflon^{™}, Parylene^{™} or metal coating also makes the composite structure, in which the base material is made of silicone, stronger than the traditional silicone band. A stronger band reduces the risk of fracture.

In one alternative to the first embodiment, the elongate composite structure is designed for mechanical adjustment, such as the mechanical solutions disclosed in International Application No. WO 01/45486. In this alternative, the property improving means comprises a core of a soft viscoelastic material, such as silicone gel, typically having a hardness less than 20 Shore, cellulose gel or collagen gel. Where silicone gel is chosen, it may be "Med 3-6300" manufactured by Nusil. Hard silicone constitutes the base material, typically having a hardness of at least 60 Shore, and covers the soft core of viscoelastic material. The soft core makes the implanted elongate composite structure less injurious to the stomach or esophagus, and reduces the injury of such organs. Furthermore, the soft core of viscoelastic material may be formed to enclose and protect mechanical adjustment components and other components of the composite structure, whereby fibrosis is prevented from growing into such components.

In another alternative to the first embodiment, the elongate composite structure is designed for hydraulic adjustment, such as the hydraulic solutions disclosed in International Application No. WO 01/50833. In this alternative, the base material forms a closed tubing, which can be inflated by adding hydraulic fluid to the interior of the tubing and deflated by withdrawing hydraulic fluid from the interior of the tubing. The coating of Teflon^{™}, Parylene^{™} or metal may cover the inner surface of the tubing. The base material may form two coaxial tubular layers of hard silicon, and the property improving means may comprise a tubular intermediate layer of a soft viscoelastic material located between the coaxial tubular layers. Alternatively, the base material may form an outer tubular layer and an inner arcuate layer attached to the outer tubular layer, the outer and inner layers defining a curved space extending longitudinally along the tubing. The property improving means may comprise a viscoelastic material filling the space. The tubing is applied around the stomach or esophagus so that the space with viscoelastic material is located closest to the stomach or esophagus. The viscoelastic material gives the advantages that erosion of the stomach or esophagus is reduced and the risk of hydraulic fluid leaking from the tubing is decreased.

The first layer of the base material preferably is comprised of hard silicone, whereas the second layer is comprised of a polyurethane layer. In a traditional silicone band, especially the tubular type, that is formed in a loop to constrict the stomach or esophagus, the inner surface of the band loop that contacts the stomach or esophagus forms bulges and creases that repeatedly change as the band is subjected to dynamic movements from the stomach or esophagus and when the size of the band is adjusted. As a consequence, the implanted traditional silicone band has the drawback that it may crack after some time due to fatigue of the silicone material. With the elongate composite structure of the invention, in which silicone and polyurethane constitute the base material and the fatigue resistant polyurethane layer covers the silicone material on the side of the elongate composite structure that contacts the stomach or esophagus, this drawback is eliminated.

The property improving means suitably comprises a coating that is coated on the base material , wherein the coating has better aggressive body fluid resistance properties and/or better anti-friction properties than hard silicone. As described above in connection with the first embodiment, the coating may comprise a Teflon^{™} or Parylene^{™} coating, or a biocompatible metal coating.

The layer of hard silicone may form an inflatable tubing and the layer of polyurethane may cover the hard silicone layer within the tubing.

In accordance with an embodiment of the invention, the base material forms an inflatable tubing and the property improving means comprises a liquid impermeable coating coated on the base material. The coating may be coated on the external and/or internal surface of the tubing. Preferably, the liquid impermeable coating comprises a Parylener^{™} coating, or a biocompatible metal coating. Where hard silicone, which is a liquid semi-permeable material, constitutes part of the base material, the coating of Parylene^{™} or metal gives the advantage that the tubing may be inflated by hydraulic fluid under pressure without risking fluid diffusing through the silicone wall of the tubing.

Also, not in accordance with the invention, the base material may form two coaxial tubular layers of hard silicon, and the property improving means may comprise a tubular intermediate layer of a soft viscoelastic material located between the coaxial tubular layers. Alternatively, the base material may form an outer tubular layer of hard silicone and an inner arcuate layer of silicone attached to the outer tubular layer. The outer and inner layers define a curved space extending longitudinally along the tubing and filled with the viscoelastic material. The tubing is intended to be applied around the stomach or esophagus so that the space with viscoelastic material is located closest to the stomach or esophagus.

In accordance with a further embodiment, the property improving means comprises gas, such as air, contained in a multiplicity of cavities formed in the base material to improve the flexibility of the composite structure. In this case, Teflon^{™} advantageously constitutes the base material. The cavities may be defined by net structures of the Teflon^{™} material. Thus, the resulting composite structure of Teflon^{™} and cavities filled with gas is strong, flexible and aggressive body fluid resistant, and has good tensile strength and good anti-friction properties. Also, in the fourth embodiment, the elongate composite structure may comprise an inflatable tubing.

The present invention also provides an implantable constriction device according to claim 1 for forming a stoma opening in the stomach or esophagus of a patient, characterized by an elongate composite structure adapted to constrict the stomach or esophagus of the patient, wherein the composite structure includes an elongate biocompatible self-supporting base material having surfaces exposed to aggressive body cells, when the constriction device is implanted in the patient, and a cell barrier coating on the surfaces to prevent body cells from breaking down the base material, which is typically silicone. If the base material were broken down by such body cells, typically macrophages or killer cells, histological particles would be spread in the human body.

The barrier coating may comprise a Parylene^{™} coating or a biocompatible metal coating.

Alternatively, the barrier coating may comprise a composite of different materials to achieve the cell-barrier protection as described above. There are several examples of such composite materials on the market, for example a composite of polyurethane and silicone called Elaston^{™}.

The invention is explained in more detail in the following with reference to the accompanying drawings, in which:
Figure 1 is a front view of a mechanical constriction device.
Figure 2 is an enlarged cross-section along the line II-II in Figure 1.
Figures 3 and 4 are modifications of the embodiment shown in Figure 2.
Figure 5 is a front view of a hydraulic constriction device.
Figure 6 is an enlarged cross-section along the line VI-VI in Figure 5.
Figures 7 -10 are modifications of the embodiment shown in Figure 6.
Figure 11 is a modification of the embodiment shown in Figure 2.

Referring to the drawing figures, like reference numerals designate identical or corresponding elements throughout the several figures.

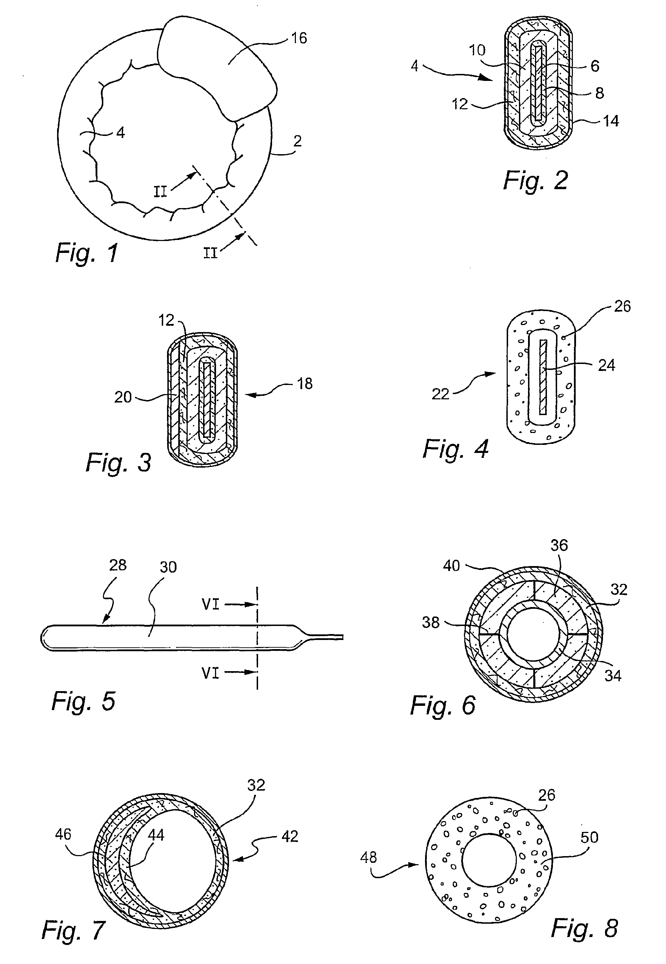

Figure 1 illustrates a mechanical constriction device 2 according to the invention comprising an elongate composite structure 4 adapted to extend around and constrict the stomach or esophagus of a patient to form a restricted stoma opening therein. Referring to Figure 2, the elongate composite structure 4 comprises a strong band 6 of nylon or the like, a tubular layer 8 of hard silicone, in which the band 6 slides, a soft layer 10 of a viscoelastic material, here a silicone gel having a hardness not more than 20 Shore, encircling the hard silicone layer 8, and a tubular layer 12 of a self-supporting base material of hard silicone having a hardness of at least 60 Shore, surrounding the soft silicon layer 10. A coating 14 of Teflon^{™}, Parylene^{™} or a biocompatible metal, such as gold, silver or titanium, is coated on the outer hard silicone layer 12 to make the composite structure resistant to aggressive body fluids and to give the composite structure good anti-friction properties. A coating of Teflon^{™}, Parylene^{™} or metal may also be coated on the internal surface of the inner tubular hard silicone layer 8 to reduce the friction between the nylon band 6 and the layer 8. The constriction device 2 has an adjustment means 16 that can displace the end portions of the nylon band 6 relative to each other to either enlarge or constrict the stoma opening.

Figure 3 shows an elongate composite structure 18 similar to that of Figure 2, except that a layer 20 of a fatigue resistant material, here polyurethane, is applied on the hard silicone layer 12 along the inner side of the structure 18 that is intended to contact the stomach or esophagus. Alternatively, the layer 20 may be tubular and surround the layer 12.

Figure 4 shows a cross-section of an elongate composite structure 22 of an embodiment in which Teflon^{™} constitutes the self-supporting base material, which is formed with a longitudinal cavity in which a strong nylon band 24 slides. Property improving means in the form of gas, here air, contained in a multiplicity of cavities 26 are formed in the base material to improve the flexibility thereof.

Figure 5 shows a hydraulic constriction device 28 comprising an elongate composite structure in the form of an inflatable tubing 30, in which the base material of hard silicone forms an outer tubular layer 32 and an inner coaxial layer 34. A viscoelastic material, here soft silicone gel, forms an intermediate layer 36 located between the tubular layers 32,34. Four longitudinal partition walls 38 between the tubular layers 32,34 divide the intermediate layer 36 into four sections to prevent the silicone gel from displacing in the circumferential direction of the tubing 30. (Also the embodiments according to Figures 2 and 3 may be provided with such longitudinal partition walls.) The outer layer 32 is coated with a coating 40 of Teflon^{™}, Parylene^{™} or metal. Also the inner layer 34 may be coated with a coating of Teflon^{™}, Paryleme^{™} or metal. If a Parylene^{™} or metal coating is chosen the composite structure will be completely liquid impermeable.

Figure 7 shows a tubing 42 similar to that of Figure 6, except that an inner arcuate layer 44 is substituted for the inner tubular layer 34. The arcuate layer 44 is attached to the outer tubular layer 32, so that the outer tubular layer 32 and the arcuate layer 44 define a curved space extending longitudinally along the tubing 42. A viscoelastic material, here silicone gel 46, fills the space. In this embodiment there is no need for partition walls of the kind shown in the embodiment according to Figure 6. The tubing 42 is intended to be applied around the stomach or esophagus so that the space with the protecting soft silicone gel 46 is located close to the stomach or esophagus.

As taught by the embodiment of Figure 7, in the composite structures shown in Figures 2 and 3 the soft silicone gel may alternatively be applied in a longitudinal space close to the inner side of the elongate composite structure 4 and 18, respectively, that is intended to contact the stomach or esophagus.

In the same manner as described above in connection with the embodiment of Figure 3, a layer of a fatigue resistant material, here polyurethane, may be applied on the outer tubular layer 32 of hard silicone of the tubing 30 and 42, respectively, along the side of the tubing 30 and 42, respectively, that is intended to contact the stomach or esophagus, when the tubing 30 and 42, respectively, encircles the stomach or esophagus.

Figure 8 shows a cross-section of an elongate composite structure 48 of an embodiment of the invention, in which Teflon^{™} constitutes the self-supporting base material, which is formed to an inflatable tubing 50. Property improving means in the form of gas contained in a multiplicity of cavities 26 are formed in the base material to improve the flexibility of the tubing 50.

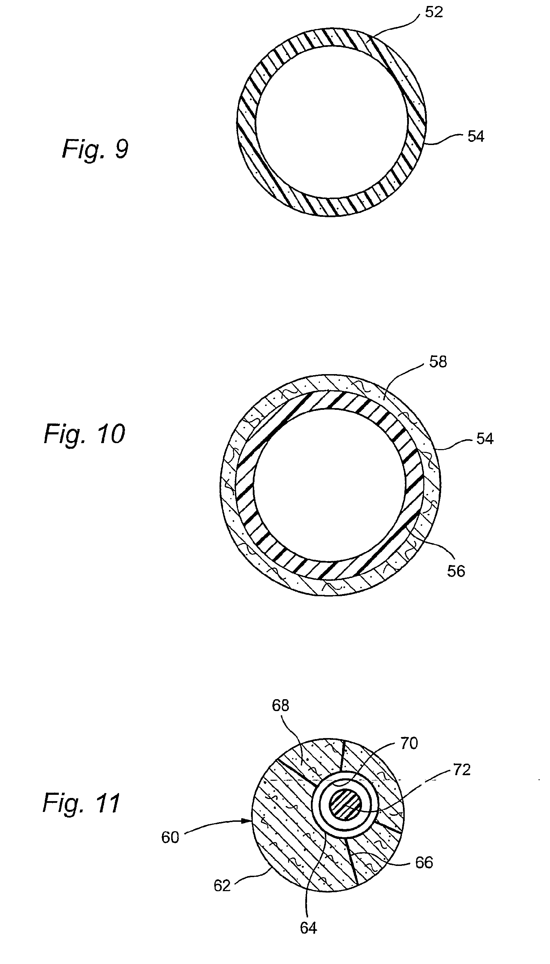

Figure 9 shows a cross section of a tubular composite structure of an embodiment of the invention, in which the self-supporting base material 52 is made of a polymer material suited for implantation, for example silicone or polyurethane. A property improving coating 54, for example made of Parylene^{™}, Teflon^{™} or metal, is applied on the external surface or on both the external and internal surfaces of the tubular structure

Figure 10 shows the same embodiment as Figure 9 except that the base material comprises a layer 56 of polyurethane surrounded by a layer 58 of silicone according to the invention.

Figure 11 shows a cross-section of a mechanical constriction device of another embodiment, comprising a double walled tubing 60 of a self-supporting base material of hard silicone. The tubing 60 has an external wall 62 and an internal wall 64 spaced from the external wall 62,partition walls 66 dividing the space between the external and internal walls 62 and 64, respectively, of the tubing 60 into longitudinal cells 68, which are filled with a soft viscoelastic material, for example silicone gel. The internal wall 64 is coated with a friction reducing coating 70, for example made of Teflon^{™} or the like. A strong band 72 of nylon or the like slides in the tubing 60 on the friction reducing coating 70 to enable adjustment of the constriction device in the same manner as described above in connection with the embodiment according to Figures 1 and 2.

Although the present invention has been described in terms of particular embodiments, it is not intended that the invention be limited to those embodiments. Modifications of the embodiments will be apparent to those skilled in the art. The scope of the invention is defined by the claims that follow.

## Claims

1. An implantable constriction device (2) for forming a restricted stoma opening in the stomach or esophagus of a patient, comprising an elongate composite structure adapted to constrict the stomach or esophagus of the patient, wherein the elongate composite structure is composed of a base material making the composite structure self-supporting, **characterized by** a property improving means (14, 20, 40, 46, 54, 70) for improving at least one physical property of the composite structure other than self-supporting properties, wherein the base material comprises a layer (56) of polyurethane and a layer (58) of silicone, wherein the property improving means comprises a layer applied on the base material, and wherein the layer applied on the base material is of a material different than the base material.

2. An implantable constriction device according to claim 1, wherein the layer of the property improving means is applied on the base material at least along a side of the elongate composite structure that is intended to contact the stomach or esophagus.

3. An implantable constriction device according to claim 2, wherein the base material forms an inflatable tubing.

4. An implantable constriction device according to claim 3, wherein the tubing has an inner surface defining the interior of the tubing, and the layer of the property improving means covers the inner surface.

5. An implantable constriction device according to claim 2, wherein the property improving means further comprises a core of a viscoelastic material covered with the self-supporting base material.

6. An implantable constriction device according to claim 2, wherein the layer of the property improving means comprises a coating coated on the base material.

7. An implantable constriction device according to claim 1, wherein the base material forms a first layer of hard silicone and a second layer of polyurethane applied on the first layer, the second layer being more fatigue resistant than the first layer.

8. An implantable constriction device according to claim 7, wherein the layer of the property improving means covers the layers of the base material along a side of the elongate composite structure that is intended to contact the stomach or esophagus.

9. An implantable constriction device according to any one of claims 1, 7 and 8, wherein the property improving means comprises a coating on the base material, the coating having better aggressive body fluid resistance properties and/or better anti-friction properties than the base material.

10. An implantable constriction device according to claim 9, wherein the coating is selected from the group consisting of Teflon^{™}, Parylene^{™}, and biocompatible metal coating.

11. An implantable constriction device according to claim 10, wherein the biocompatible metal coating is selected from the group consisting of gold, silver and titanium.

12. An implantable constriction device according to claim 7, wherein the base material forms an inflatable tubing, and the layer of the property improving means covers the base material within the tubing.

13. An implantable constriction device according to claim 1, wherein the tubing has an external surface of the base material and an internal surface of the base material defining the interior of the tubing, the layer of the property improving means being applied on the external surface and/or internal surface.

14. An implantable constriction device according to claim 1 or 13, wherein the layer of the property improving means is a coating coated on the base material, the coating being selected from the group consisting of Parylene^{™}, Teflon^{™}, and a biocompatible metal coating.

15. An implantable constriction device according to claim 14, wherein the biocompatible metal coating is selected from the group consisting of gold, silver and titanium.

16. An implantable constriction device according to any one of claims 1-15, wherein hard silicone and polyurethane constitute the base material.

17. An implantable constriction device according to claim 3, wherein the base material forms two coaxial tubular layers and the layer of the property improving means consists of a tubular intermediate protection layer of a soft viscoelastic material located between the coaxial tubular layers.

18. An implantable constriction device according to claim 3, wherein the base material forms an outer tubular layer and an inner arcuate layer attached to the outer tubular layer, the outer and inner layers defining a curved space extending longitudinally along the tubing, and the property improving means consists of a protection layer of viscoelastic material filling the space.

19. An implantable constriction device according to any one of claims 5, 17 and 18, wherein the viscoelastic material is selected from the group consisting of silicone gel, cellulose gel, and collagen gel.

20. An implantable constriction device according to claim 5, wherein the layer of the property improving means comprises a coating coated on the base material, the coating being selected from the group consisting of Teflon^{™} and Parylene^{™}.

21. An implantable constriction device according to claim 1, wherein the elongate composite structure is non-inflatable.

22. An implantable constriction device according to claim 21, further comprising an adjustment means adapted to mechanically adjust the non-inflatable composite structure to change the constriction of the stomach or esophagus.

23. An implantable constriction device according to any one of the preceding claims, wherein the elongate composite structure is adapted to externally constrict the stomach or esophagus.

24. An implantable constriction device according to claim 1, wherein the layer of the property improving means comprises a cell barrier coating coated on the surfaces to prevent body cells from breaking down the base material.

25. An implantable constriction device according to claim 1, wherein the layer of the property improving means comprises a liquid impermeable coating coated on the base material.

26. An implantable constriction device according to claim 1, wherein the layer of the property improving means is more fatigue resistant than the base material.

27. An implantable constriction device according to claim 1, 9, 17, and 24-26, wherein the base material forms at least two tubular layers.

28. An implantable constriction device according to claim 27, wherein the layer of the property improving means is applied externally on the tubular layers of the base material.

29. An implantable constriction device according to claim 27, wherein the layer of the property improving means is applied internally on the tubular layers of the base material.

30. An implantable constriction device according to claim 27, wherein the property improving means comprises a first layer applied externally on the tubular base material and a second layer applied internally on the tubular layers of the base material.

31. An implantable constriction device according to claim 27, wherein the composite structure comprises an external tubular layer of the base material and an internal tubular layer of the base material extending inside of and being spaced from the external tubular layer, whereby the external and internal tubular layers define a space, the layer of the property improving means extending in the space.

32. An implantable constriction device according to any one of claims 9, 17, and 24-26, wherein the layer of the property improving means is a coating coated on the base material, the coating being selected from the group consisting of Parylene^{™}, Teflon^{™}, and a biocompatible metal coating.

33. An implantable constriction device according to claim 32, wherein the biocompatible metal coating is selected from the group consisting of gold, silver and titanium.

## Patentansprüche

1. Implantierbare Einschnüreinrichtung (2) zum Bilden einer eingeschränkten Öffnung in dem Magen oder der Speiseröhre eines Patienten, umfassend eine längliche Verbundstruktur, ausgebildet zum Einschnüren des Magens oder der Speiseröhre des Patienten, wobei die längliche Verbundstruktur sich aus einem die Verbundstruktur selbsttragend machenden Basismaterial zusammensetzt, **gekennzeichnet durch** eine Eigenschaft-verbessernde Einrichtung (14, 20, 40, 46, 54, 70) zum Verbessern mindestens einer weiteren physikalischen Eigenschaft der Verbundstruktur außer den selbsttragenden Eigenschaften, wobei das Basismaterial eine Schicht (56) aus Polyurethan und eine Schicht (58) aus Silikon aufweist, wobei die Eigenschaften-verbessernde Einrichtung ein auf das Basismaterial aufgebrachte Schicht aufweist, und wobei die auf das Basismaterial aufgebrachte Schicht ein von dem Basismaterial verschiedenes Material ist.

2. Implantierbare Einschnüreinrichtung nach Anspruch 1, bei der die Schicht der Eigenschaft-verbessernden Einrichtung auf das Basismaterial zumindest entlang einer Seite der länglichen Verbundstruktur aufgebracht ist, die für die Berührung mit dem Magen oder der Speiseröhre vorgesehen ist.

3. Implantierbare Einschnüreinrichtung nach Anspruch 2, bei der das Basismaterial einen aufblasbaren Schlauchkörper bildet.

4. Implantierbare Einschnüreinrichtung nach Anspruch 3, bei der der Schlauchkörper eine Innenfläche aufweist, die das Innere des Schlauchkörpers definiert, und die Schicht der Eigenschaft-verbessernden Einrichtung die Innenfläche bedeckt.

5. Implantierbare Einschnüreinrichtung nach Anspruch 2, bei der die Eigenschaft-verbessernde Einrichtung außerdem einen Kern aus einem viskoelastischem Material enthält, der mit dem selbsttragenden Basismaterial bedeckt ist.

6. Implantierbare Einschnüreinrichtung nach Anspruch 2, bei der die Schicht der Eigenschaft-verbessernden Einrichtung ein auf das Basismaterial aufgebrachten Überzug aufweist.

7. Implantierbare Einschnüreinrichtung nach Anspruch 1, bei der das Basismaterial eine erste Schicht aus Hartsilikon und eine zweite Schicht aus auf die erste Schicht aufgebrachtem Polyurethan aufweist, wobei die zweite Schicht ermüdungsbeständiger als die erste Schicht ist.

8. Implantierbare Einschnüreinrichtung nach Anspruch 7, bei der die Schicht der Eigenschaft-verbessernden Einrichtung die Schichten des Basismaterials entlang einer Seite der länglichen Verbundstruktur bedeckt, die für die Berührung mit dem Magen oder der Speiseröhre vorgesehen ist.

9. Implantierbare Einschnüreinrichtung nach einem der Ansprüche 1, 7 und 8, bei der die Eigenschaft-verbessernde Einrichtung einen Überzug auf dem Basismaterial aufweist, welcher bessere Widerstandsfähigkeit gegenüber aggressivem Körperfluid und/oder bessere Antireibeigenschaften als das Basismaterial aufweist.

10. Implantierbare Einschnüreinrichtung nach Anspruch 9, bei der der Überzug ausgewählt ist aus der Gruppe Teflon®, Parylene® und einem biokompatiblen Metallüberzug.

11. Implantierbare Einschnüreinrichtung nach Anspruch 10, bei der der biokompatible Metallüberzug ausgewählt ist aus der Gruppe Gold, Silber und Titan.

12. Implantierbare Einschnüreinrichtung nach Anspruch 7, bei der das Basismaterial einen aufblasbaren Schlauchkörper bildet und die Schicht der Eigenschaft-verbessernden Einrichtung das Basismaterial innerhalb des Schlauchkörpers bedeckt.

13. Implantierbare Einschnüreinrichtung nach Anspruch 1, bei der der Schlauchkörper eine Außenoberfläche des Basismaterial aufweist und die Innenfläche des Basismaterials das Innere des Schlauchkörpers definiert, wobei die Schicht der Eigenschaft-verbessernden Einrichtung auf die Außenfläche und/oder die Innenfläche aufgebracht ist.

14. Implantierbare Einschnüreinrichtung nach Anspruch 1 oder 13, bei der die Schicht der Eigenschaft-verbessernden Einrichtung ein auf das Basismaterial aufgebrachter Überzug ist, wobei der Überzug ausgewählt ist aus der Gruppe Paryleine®, Teflon® und einem biokompatiblen Metallüberzug.

15. Implantierbare Einschnüreinrichtung nach Anspruch 14, bei der der biokompatible Metallüberzug ausgewählt ist aus der Gruppe Gold, Silber und Titan.

16. Implantierbare Einschnüreinrichtung nach einem der Ansprüche 1 bis 15, bei der Hartsilikon und Polyurethan das Basismaterial bilden.

17. Implantierbare Einschnüreinrichtung nach Anspruch 3, bei der das Basismaterial zwei koaxiale Schlauchschichten bildet und die Schicht der Eigenschaft-verbessernden Einrichtung aus einer schlauchförmigen Zwischen-Schutzschicht eines weich-viskoelastischen Materials besteht, die sich zwischen den koaxialen Schlauchschichten befindet.

18. Implantierbare Einschnüreinrichtung nach Anspruch 3, bei der das Basismaterial eine äußere Schlauchschicht und eine an der äußeren Schlauchschicht befestigte innere bogenförmige Schicht bildet, und die äußere und die innere Schicht einen gekrümmten Raum definieren, der sich entlang dem Schlauchkörper in Längsrichtung erstreckt und die Eigenschaft-verbessernde Einrichtung aus einer Schutzschicht aus viskoelastischem Material besteht, die den Raum ausfüllt.

19. Implantierbare Einschnüreinrichtung nach einem der Ansprüche 5, 17 und 18 bei der das viskoelastische Material ausgewählt ist aus der Gruppe Silikon-Gel, Zellulose-Gel und Kollagen-Gel.

20. Implantierbare Einschnüreinrichtung nach Anspruch 5, bei der die Schicht der Eigenschaft-verbessernden Einrichtung einen auf das Basismaterial aufgebrachten Überzug aufweist, der ausgewählt ist aus der Gruppe Teflon® und Parylene®.

21. Implantierbare Einschnüreinrichtung nach Anspruch 1, bei der die längliche Verbundstruktur nicht-aufblasbar ist.

22. Implantierbare Einschnüreinrichtung nach Anspruch 21, weiterhin umfassend eine Einstelleinrichtung, ausgebildet zum mechanischen Einstellen der nicht-aufblasbaren Verbundstruktur, um die Einschnürung des Magens oder der Speiseröhre zu ändern.

23. Implantierbare Einschnüreinrichtung nach einem der vorhergehenden Ansprüche, bei der die längliche Verbundstruktur dazu ausgebildet ist, den Magen oder die Speiseröhre von außen einzuschnüren.

24. Implantierbare Einschnüreinrichtung nach Anspruch 1, bei der die Schicht der Eigenschaft-verbessernden Einrichtung einen Zell-Barrieren-Überzug aufweist, der auf die Oberflächen aufgebracht ist, um zu verhindern, dass Körperzellen das Basismaterial zerstören.

25. Implantierbare Einschnüreinrichtung nach Anspruch 1, bei der die Schicht der Eigenschaft-verbessernden Einrichtung einen auf das Basismaterial aufgebrachten flüssigkeits-undurchlässigen Überzug aufweist.

26. Implantierbare Einschnüreinrichtung nach Anspruch 1, bei der die Schicht der Eigenschaft-verbessernden Einrichtung ermüdungsbeständiger als das Basismaterial ist.

27. Implantierbare Einschnüreinrichtung nach Anspruch 1, 9, 17 und 24 bis 26, bei der das Basismaterial mindestens zwei Schlauchschichten bildet.

28. Implantierbare Einschnüreinrichtung nach Anspruch 27, bei der die Schicht der Eigenschaft-verbessernden Einrichtung außen auf die Schlauchschichten des Basismaterials aufgebracht ist.

29. Implantierbare Einschnüreinrichtung nach Anspruch 27, bei der die Schicht der Eigenschaft-verbessernden Einrichtung innen auf die Schlauchschichten des Basismaterials aufgebracht ist.

30. Implantierbare Einschnüreinrichtung nach Anspruch 27, bei der die Eigenschaft-verbessernde Einrichtung eine erste Schicht aufweist, die außen auf das schlauchförmige Basismaterial aufgebracht ist, außerdem eine zweite Schicht, die innen auf die Schlauchschichten des Basismaterials aufgebracht ist.

31. Implantierbare Einschnüreinrichtung nach Anspruch 27, bei der die Verbundstruktur eine äußere Schlauchschicht des Basismaterials und eine innere Schlauchschicht des Basismaterials aufweist, wobei letztere sich im Inneren der äußeren Schlauchschicht erstreckt und von dieser beabstandet ist, wodurch die äußere und innere Schlauchschichten einen Raum definieren, in welchem sich die Schicht der Eigenschaft-verbessernden Einrichtung erstreckt.

32. Implantierbare Einschnüreinrichtung nach einem der Ansprüche 9, 17 und 24 bis 26, bei der die Schicht der Eigenschaft-verbessernden Einrichtung ein auf das Basismaterial aufgebrachte Überzug ist, ausgewählt aus der Gruppe Parylene®, Teflon® und einem biokompatiblen Metallüberzug.

33. Implantierbare Einschnüreinrichtung nach Anspruch 32, bei der der biokompatible Metallüberzug ausgewählt ist aus der Gruppe Gold, Silber und Titan.

## Revendications

1. Dispositif (2) de constriction implantable destiné à former un orifice de stomie restreint dans l'estomac ou l'oesophage d'un patient, comprenant une structure composite allongée conçue pour resserrer l'estomac ou l'oesophage du patient, dans lequel la structure composite allongée est constituée d'une matière de base rendant la structure composite autoportante, **caractérisé par** un moyen (14, 20, 40, 46, 54, 70) d'amélioration de propriété destiné à améliorer au moins une propriété physique de la structure composite autre que les propriétés d'autoportance, dans lequel la matière de base comprend une couche (56) de polyuréthane et une couche (58) de silicone, dans lequel le moyen d'amélioration de propriété comprend une couche appliquée sur la matière de base, et dans lequel la couche appliquée sur la matière de base est constituée d'une matière différente de la matière de base.

2. Dispositif de constriction implantable selon la revendication 1, dans lequel la couche du moyen d'amélioration de propriété est appliquée sur la matière de base au moins le long d'un côté de la structure composite allongée qui est destinée à entrer en contact avec l'estomac ou l'oesophage.

3. Dispositif de constriction implantable selon la revendication 2, dans lequel la matière de base forme une gaine gonflable.

4. Dispositif de constriction implantable selon la revendication 3, dans lequel la gaine présente une surface intérieure définissant l'intérieur de la gaine, et la couche du moyen d'amélioration de propriété recouvre la surface intérieure.

5. Dispositif de constriction implantable selon la revendication 2, dans lequel le moyen d'amélioration de propriété comprend en outre un coeur de matière viscoélastique recouvert par la matière de base autoportante.

6. Dispositif de constriction implantable selon la revendication 2, dans lequel la couche du moyen d'amélioration de propriété comprend un revêtement déposé sur la matière de base.

7. Dispositif de constriction implantable selon la revendication 1, dans lequel la matière de base forme une première couche de silicone dur et une deuxième couche de polyuréthane appliquée sur la première couche, la deuxième couche étant plus résistante à la fatigue que la première couche.

8. Dispositif de constriction implantable selon la revendication 7, dans lequel la couche du moyen d'amélioration de propriété recouvre les couches de la matière de base le long d'un côté de la structure composite allongée qui est destinée à entrer en contact avec l'estomac ou l'oesophage.

9. Dispositif de constriction implantable selon l'une des revendications 1, 7 et 8, dans lequel le moyen d'amélioration de propriété comprend un revêtement sur la matière de base, le revêtement présentant de meilleures propriétés de résistance aux fluides corporels agressifs et/ou de meilleures propriétés antifriction que la matière de base.

10. Dispositif de constriction implantable selon la revendication 9, dans lequel le revêtement est sélectionné parmi le groupe composé de Téflon^{™}, de Parylène^{™} et de revêtement métallique biocompatible.

11. Dispositif de constriction implantable selon la revendication 10, dans lequel le revêtement métallique biocompatible est sélectionné parmi le groupe composé d'or, d'argent et de titane.

12. Dispositif de constriction implantable selon la revendication 7, dans lequel la matière de base forme une gaine gonflable, et la couche du moyen d'amélioration de propriété recouvre la matière de base à l'intérieur de la gaine.

13. Dispositif de constriction implantable selon la revendication 1, dans lequel la gaine présente une surface extérieure de la matière de base et une surface intérieure de la matière de base définissant l'intérieur de la gaine, la couche du moyen d'amélioration de propriété étant appliquée sur la surface extérieure et/ou la surface intérieure.

14. Dispositif de constriction implantable selon la revendication 1 ou 13, dans lequel la couche du moyen d'amélioration de propriété est un revêtement déposé sur la matière de base, le revêtement étant sélectionné parmi le groupe composé de Parylène^{™}, de Téflon^{™} et d'un revêtement métallique biocompatible.

15. Dispositif de constriction implantable selon la revendication 14, dans lequel le revêtement métallique biocompatible est sélectionné parmi le groupe composé d'or, d'argent et de titane.

16. Dispositif de constriction implantable selon l'une des revendications 1 à 15, dans lequel le silicone dur et le polyuréthane constituent la matière de base.

17. Dispositif de constriction implantable selon la revendication 3, dans lequel la matière de base forme deux couches tubulaires coaxiales et la couche du moyen d'amélioration de propriété se compose d'une couche de protection intermédiaire tubulaire de matière viscoélastique souple située entre les couches tubulaires coaxiales.

18. Dispositif de constriction implantable selon la revendication 3, dans lequel la matière de base forme une couche tubulaire extérieure et une couche arquée intérieure fixée à la couche tubulaire extérieure, les couches extérieure et intérieure définissant un espace incurvé s'étendant longitudinalement le long de la gaine, et le moyen d'amélioration de propriété se compose d'une couche de protection de matière viscoélastique remplissant l'espace.

19. Dispositif de constriction implantable selon l'une des revendications 5, 17 et 18, dans lequel la matière viscoélastique est sélectionnée parmi le groupe composé de gel de silicone, de gel de cellulose et de gel de collagène.

20. Dispositif de constriction implantable selon la revendication 5, dans lequel la couche du moyen d'amélioration de propriété comprend un revêtement déposé sur la matière de base, le revêtement étant sélectionné parmi le groupe composé de Téflon^{™} et de Parylène^{™}.

21. Dispositif de constriction implantable selon la revendication 1, dans lequel la structure composite allongée est non-gonflable.

22. Dispositif de constriction implantable selon la revendication 21, comprenant en outre un moyen de réglage conçu pour régler mécaniquement la structure composite non-gonflable afin de modifier la constriction de l'estomac ou de l'oesophage.

23. Dispositif de constriction implantable selon l'une des revendications précédentes, dans lequel la structure composite allongée est conçue pour resserrer de l'extérieur l'estomac ou l'oesophage.

24. Dispositif de constriction implantable selon la revendication 1, dans lequel la couche du moyen d'amélioration de propriété comprend un revêtement barrière de cellules déposé sur les surfaces pour empêcher les cellules corporelles de briser la matière de base.

25. Dispositif de constriction implantable selon la revendication 1, dans lequel la couche du moyen d'amélioration de propriété comprend un revêtement imperméable liquide déposé sur la matière de base.

26. Dispositif de constriction implantable selon la revendication 1, dans lequel la couche du moyen d'amélioration de propriété est plus résistante à la fatigue que la matière de base.

27. Dispositif de constriction implantable selon la revendication 1, 9, 17 et 24 à 26, dans lequel la matière de base forme au moins deux couches tubulaires.

28. Dispositif de constriction implantable selon la revendication 27, dans lequel la couche du moyen d'amélioration de propriété est appliquée à l'extérieur sur les couches tubulaires de la matière de base.

29. Dispositif de constriction implantable selon la revendication 27, dans lequel la couche du moyen d'amélioration de propriété est appliquée à l'intérieur sur les couches tubulaires de la matière de base.

30. Dispositif de constriction implantable selon la revendication 27, dans lequel le moyen d'amélioration de propriété comprend une première couche appliquée à l'extérieur sur la matière de base tubulaire et une deuxième couche appliquée à l'intérieur sur les couches tubulaires de la matière de base.

31. Dispositif de constriction implantable selon la revendication 27, dans lequel la structure composite comprend une couche tubulaire extérieure de la matière de base et une couche tubulaire intérieure de la matière de base s'étendant à l'intérieur de, et étant espacée de la couche tubulaire extérieure, par laquelle les couches tubulaires extérieure et intérieure définissent un espace, la couche du moyen d'amélioration de propriété s'étendant dans l'espace.

32. Dispositif de constriction implantable selon l'une des revendications 9, 17 et 24 à 26, dans lequel la couche du moyen d'amélioration de propriété est un revêtement déposé sur la matière de base, le revêtement étant sélectionné parmi le groupe composé de Parylène^{™}, de Téflon^{™} et d'un revêtement métallique biocompatible.

33. Dispositif de constriction implantable selon la revendication 32, dans lequel le revêtement métallique biocompatible est sélectionné parmi le groupe composé d'or, d'argent et de titane.
